# EUROPEAN PATENT APPLICATION

(11) **EP 1 529 486 A2**
(43) Date of publication of application: **11.05.2005**
(21) Application number: 05002507.1
(22) Date of filing: 11.02.2004
(51) Int. Cl.: A61B 5/00, A61B 5/117, G06F 19/00, G06F 159/00

(54) **Storage device and biological data acquiring apparatus, and a data transmitter**

(30) Priority: 28.02.2003 JP 2003052410; 30.06.2003 JP 2003186271
(62) Divisional of application: 04003030.6
(71) Applicant: TANITA CORPORATION, Tokyo 174-8630 (JP)
(72) Inventor: Yoshizawa, Masaki, Tokyo (JP); Miyashita, Yuichi, Tokyo (JP)
(74) Representative: Grünecker, Kinkeldey, Stockmair & Schwanhäusser Anwaltssozietät

(57) **Abstract**

A storage device for a biological data acquiring apparatus is independent and detachable from the main unit of the biological data acquiring apparatus which incorporates biological data acquiring means for acquiring biological data and has biological data storing means for storing the biological data acquired by the biological data acquiring means. Thus, the storage device can be detached from the main unit of the biological data acquiring apparatus with the biological data stored in the biological data storing means and can be connected to a data processing terminal so as to transmit the stored biological data to the data processing terminal.

The storage device has a data transmitter which carries out data transmission with a host device via a connection terminal that accommodates a predetermined data transmission standard and which comprises data signal processing means for processing a number of different data signals including a data signal that accommodates the predetermined data transmission standard and data signal selecting means for selecting a data signal to be processed by the data signal processing means.

## Description

### BACKGROUND OF THE INVENTION

### (i) Field of the Invention

The present invention relates to a biological data acquiring apparatus for acquiring biological data of a user, particularly, to a storage device for a biological data acquiring apparatus and an biological data acquiring apparatus using the storage device.

Further, the present invention relates to a data transmitter which carries out data transmission with a host device via a connection terminal which accommodates a predetermined data transmission standard, a storage device having the.data transmitter, and a biological data acquiring apparatus using the storage device having the data transmitter.

### (ii) Description of the Related Art

A variety of biological data acquiring apparatuses for acquiring biological data of a user such as a body weight, a body fat percentage, a pulse and the number of steps have been developed and actually employed. These biological data acquiring apparatuses include one which directly measures a body weight as biological data of a user in a manner similar to a scale and one which acquires desired biological data such as a body fat percentage, a body water percentage, a visceral fat level, a muscle mass, a bone density and a basal metabolic rate by performing computations based on a body weight and a bioelectrical impedance value which are measured biological data of a user and biological data such as a height, age and sex which are input by the user (refer to Patent Publications 1 and 2, for example).

Further, in recent years, there are systems that acquire desired biological data that are difficult to acquire by the biological data acquiring apparatuses alone, such as histories of changes in the biological data over a long continuous time period, by transmitting the biological data acquired by the above biological data acquiring apparatuses to a data processing terminal such as a personal computer and storing the data in the terminal or by performing such computations as described above or more complicated computations. As such a system, for example, a system is proposed which acquires biological data such as the number of steps or a waking pace of a user by use of a body motion sensor and a computation unit which are incorporated in the main body of a pedometer, transmits the biological data to a personal computer by use of a communication cable or an optical communication unit, and performs computations based on the biological data and other biological data input in the personal computer such as the height, body weight, age and sex of the user so as to acquire biological data such as a consumed calorie and a history of change thereof (refer to Patent Publication 3).

Meanwhile, as a storage device for storing data, a so-called USB (Universal Serial Bus) memory is known which is capable of exchanging data with a host device such as a personal computer via a connection terminal which accommodates the USB standard (hereinafter referred to as "USB terminal") (refer to Patent Publication 4, for example). As exemplified in Fig. 14, the conventional USB memory A has a USB terminal C for connecting to a USB terminal (not shown) of a host device and incorporates a data storing memory M such as a flash memory or EEPROM and a microprocessor COM for controlling and processing of a data signal to be transmitted and storing of data in the memory M. These USB terminal C, microprocessor COM and memory M are connected together by data buses D.
Patent Publication 1
   Japanese Patent Publication No. 5-49050
Patent Publication 2
   Japanese Patent Publication No. 2001-70273
Patent Publication 3
   Japanese Patent Publication No. 2000-41953
Patent Publication 4
   Japanese Patent Publication No. 2002-41247

The above conventional biological data acquiring apparatus has the following problems in an attempt to acquire desired biological data by transmitting biological data to a data processing terminal such as a personal computer and storing the biological data in the data processing terminal or performing computations in the data processing terminal.

Firstly, when the biological data acquiring apparatus is connected to the data processing terminal by use of a cable or the like so as to transmit the biological data to the data processing terminal, they must be placed within such an area that the cable can reach both the apparatus and the terminal. Particularly, when both the apparatus and the terminal are generally used in a stationary position (i.e., the stationary devices) as in the case where biological data is transmitted from a scale or such a body fat monitor as disclosed in the above Japanese Patent Publication No. 5-49050 to a so-called desktop type personal computer, a sufficiently long cable must be used or one of the devices must be moved to a position close to the other device upon transmission and reception of the biological data. In general households, a scale and a body fat monitor are often placed in a different room from a room in which a personal computer is set. Thus, using a long cable or moving the devices is not very practical and makes the devices difficult to use. Further, in the case of a biological data acquiring apparatus having a weight sensor such as a scale, an undesired load may be physically applied on the weight sensor depending on the position or state of connection of the cable, thereby degrading the accuracy of measurement. Meanwhile, when one of the biological data acquiring apparatus or the data processing terminal is small and can be carried easily (i.e., portable) as in the case of the pedometer disclosed in the above Japanese Patent Application Laid-Open No. 2000-41953, the possibility of occurrence of the above problem is small. However, in this case as well, handling of a cable is still cumbersome, and it can be hardly said that the devices are easy to use.

Further, when the biological data acquiring apparatus is connected to the data processing terminal in a wireless manner through optical communication such as infrared radiation, communication cannot be made when an object exists between communication units provided to the biological data acquiring apparatus and the data processing terminal. Thus, as in the case of the above connection using a cable, the positions of the devices are limited or the devices must be moved. Accordingly, the wireless connection is also not suitable for the stationary devices. Meanwhile, when an electric wave is used for carrying out the wireless connection, communication is still possible even if an object of a certain size exists, so that the above problems regarding the positions and moving of the devices are nearly completely solved. However, since communication units using electric waves are expensive, the biological data acquiring apparatus and the data processing terminal become expensive. Further, since such communication units consume a large quantity of electric currents, the useful life of a battery used in the biological data acquiring apparatus which is often battery-driven is short, and running costs are high.

Further, in data transmission based on the USB standard (hereinafter referred to as "USB transmission"), although it is possible to send and/or receive data signals at high speed, it has a problem that a CPU of relatively high performance must be provided in a host device so as to perform data signal processing. For this reason, the above USB memory can be used easily in a device incorporating a high-performance CPU such as a personal computer, and when the USB memory is used in a device without a high-performance CPU such as a biological data acquiring apparatus, a high-performance CPU must be installed in the device so as to enable the USB transmission. However, installation of the high-performance CPU in a device such as the biological data acquiring apparatus causes a significant increase in costs and is therefore unpractical.

Thus, in order to use the above USB memory in a device equipped with an inexpensive CPU such as the biological data acquiring apparatus, it is conceivable to use data transmission based on a standard such as RS-232C or data transmission based on a standard set in advance between the device and the USB memory, either of which does not require as high-performance of a CPU as the USB standard does (hereinafter collectively referred to as "serial transmission"), for data transmission between the device and the USB memory. However, in this case, a connection terminal for serial transmission must be additionally provided in the USB memory. As a result, the circuit configuration of the USB memory becomes complicated, thereby increasing the possibility of malfunctions by noises such as static electricity and electromagnetic waves, and the costs of components are also increased due to an increase in the number of components, thereby increasing the possibility of occurrences of production failures, resulting in an increase in production costs.

The above problems arise not only when the USB memory is used as a part of a biological data acquiring apparatus so as to send biological data to a data processing terminal such as a personal computer, but when a data transmitter which accommodates a predetermined data transmission standard is desired to be connected to a host device which has no data transmission capability to accommodate the predetermined data transmission standard.

The present invention has been conceived by focusing on the above conventional problems. An object of the present invention is to provide a storage device for a biological data acquiring apparatus which makes possible transmission of biological data from the biological data acquiring apparatus to a data processing terminal without use of a cable, infrared radiation, electric wave or the like when the biological data is to be transmitted from the biological data acquiring apparatus to the data processing terminal and computations are to be performed in the data processing terminal based on the biological data so as to eventually acquire desired biological data, and which can be implemented at a relatively low cost.

Another object of the present invention is to provide a biological data acquiring apparatus which can transmit biological data to a data processing terminal without use of a cable, infrared radiation, electric wave or the like when the biological data is to be transmitted from the biological data acquiring apparatus to the data processing terminal and computations are to be performed in the data processing terminal based on the biological data so as to eventually acquire desired biological data, and which can be implemented at a relatively low cost.

Still another object of the present invention is to provide a data transmitter which carries out data transmission with a host device via a connection terminal which accommodates a predetermined data transmission standard, wherein the data transmission can be carried out even with a host device which does not accommodate the predetermined data transmission standard without increasing the number of the connection terminal; a storage device having such a data transmitter; and a biological data acquiring apparatus using such a storage device.

### SUMMARY OF THE INVENTION

A storage device for a biological data acquiring apparatus according to the present invention is independent and detachable from the main unit of the biological data acquiring apparatus which incorporates biological data acquiring means for acquiring biological data and has biological data storing means for storing the biological data acquired by the biological data acquiring means.

Further, the storage device of the present invention has program software for performing computations based on the biological data stored in the biological data storing means.

Alternatively, the storage device of the present invention has program software for performing computations based on the biological data stored in the biological data storing means and an operation element for executing the program software.

Further, the storage device of the present invention has a data transmitter which carries out data transmission with a host device via a connection terminal that accommodates a predetermined data transmission standard and which comprises data signal processing means for processing a number of different data signals including a data signal that accommodates the predetermined data transmission standard and data signal selecting means for selecting a data signal to be processed by the data signal processing means.

Further, in the storage device of the present invention, the data signal selecting means selects the data signal based on a given identification signal transmitted from the host device.

Further, in the storage device of the present invention, the predetermined data transmission standard is the USB standard.

Further, the main unit of the biological data acquiring apparatus using the storage device of the present invention is stationary.

Alternatively, a biological data acquiring apparatus according to the present invention comprises a main unit and a storage device, wherein the main unit incorporates biological data acquiring means for acquiring biological data, and the storage device is independent and detachable from the main unit and has biological data storing means for storing the biological data acquired by the biological data acquiring means.

Further, in the biological data acquiring apparatus of the present invention, the storage device has program software for performing computations based on the biological data stored in the biological data storing means.

Alternatively, the storage device in the biological data acquiring apparatus of the present invention has program software for performing computations based on the biological data stored in the biological data storing means and an operation element for executing the program software.

Further, in the biological data acquiring apparatus of the present invention, the storage device has a data transmitter which carries out data transmission with a host device via a connection terminal that accommodates a predetermined data transmission standard and which comprises data signal processing means for processing a number of different data signals including a data signal that accommodates the predetermined data transmission standard and data signal selecting means for selecting a data signal to be processed by the data signal processing means.

Further, in the biological data acquiring apparatus of the present invention, the data signal selecting means selects the data signal based on a given identification signal transmitted from the host device.

Further, in the biological data acquiring apparatus of the present invention, the predetermined data transmission standard is the USB standard.

Further, the main unit of the biological data acquiring apparatus of the present invention is stationary.

Alternatively, a data transmitter according to the present invention is a data transmitter which carries out data transmission with a host device via a connection terminal that accommodates a predetermined data transmission standard and which comprises data signal processing means for processing a number of different data signals including a data signal that accommodates the predetermined data transmission standard and data signal selecting means for selecting a data signal to be processed by the data signal processing means.

The data signal selecting means selects the data signal based on a given identification signal transmitted from the host device. Further, the predetermined data transmission standard is the USB standard.

Further, a storage device according to the present invention is a storage device comprising the data transmitter according to the above present invention and a memory for storing data.

In addition, a biological data acquiring apparatus according to the present invention is a biological data acquiring apparatus comprising a main unit which incorporates biological data acquiring means for acquiring biological data and a storage device which is independent from the main unit and detachable from the main unit via a terminal for data transmission and comprises the data transmitter according to the above present invention and a memory for storing the biological data acquired by the main unit.

Further, in the biological data acquiring apparatus of the present invention, the storage device has program software for performing computations based on the biological data stored in the memory.

Alternatively, the storage device in the biological data acquiring apparatus of the present invention has program software for performing computations based on the biological data stored in the memory and an operation element for executing the program software.

Further, the main unit of the biological data acquiring apparatus of the present invention is stationary.

### BRIEF DESCRIPTION OF THE DRAWINGS

Fig. 1 is an external view of a storage device for a biological data acquiring apparatus according to the present invention.
Fig. 2 is an external view of the biological data acquiring apparatus according to the present invention.
Fig. 3 is a schematic diagram showing electronic circuit boards incorporated in the biological data acquiring apparatus according to the present invention.
Fig. 4 is a flowchart showing a flow in using the biological data acquiring apparatus according to the present invention.
Fig. 5A is a flowchart showing the data storing operation of the storage device according to the present invention.
Fig. 5B is a flowchart showing the data transmitting operation of the storage device according to the present invention.
Fig. 6 is an external view of a biological data acquiring apparatus according to the present invention together with a data processing terminal.
Fig. 7 is a schematic diagram showing the circuit configuration of a storage device according to the present invention.
Fig. 8 is a flowchart showing a first program which is executed in the storage device according to the present invention.
Fig. 9 is a diagram showing a signal in a data bus at the time of execution of the first program of Fig. 8.
Fig. 10 is a flowchart showing a second program which is executed in the storage device according to the present invention.
Fig. 11 is a diagram showing a signal in the data bus at the time of execution of the second program of Fig. 10.
Fig. 12 is a flowchart showing a control program which is executed in the main unit of the biological data acquiring apparatus according to the present invention.
Fig. 13 is a flowchart showing a subroutine in the control program of Fig. 12.

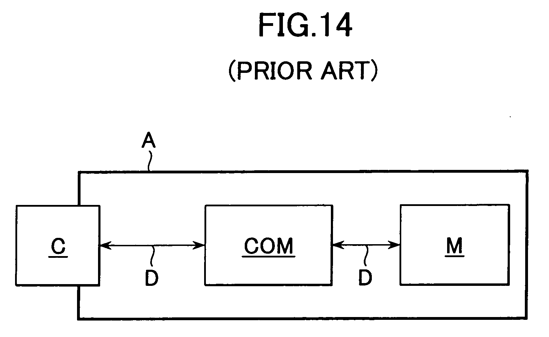

Fig. 14 is a schematic diagram showing the circuit configuration of a conventional USB memory.

### DETAILED DESCRIPTION OF THE PREFERRED EMBODIMENTS

### [Example 1]

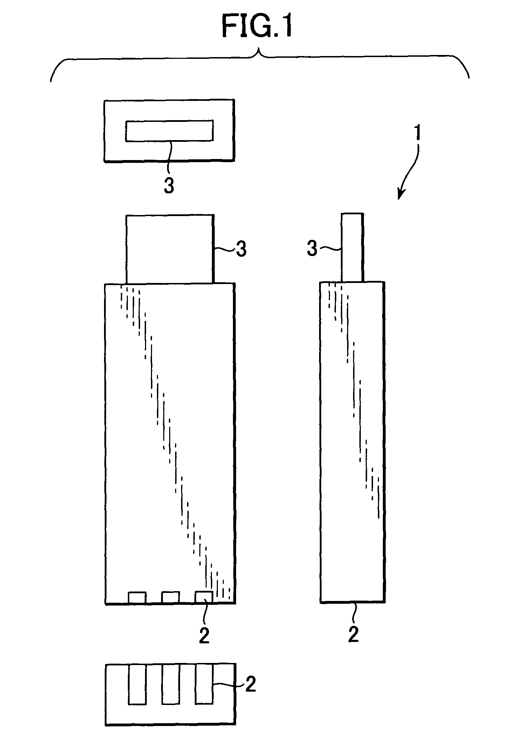

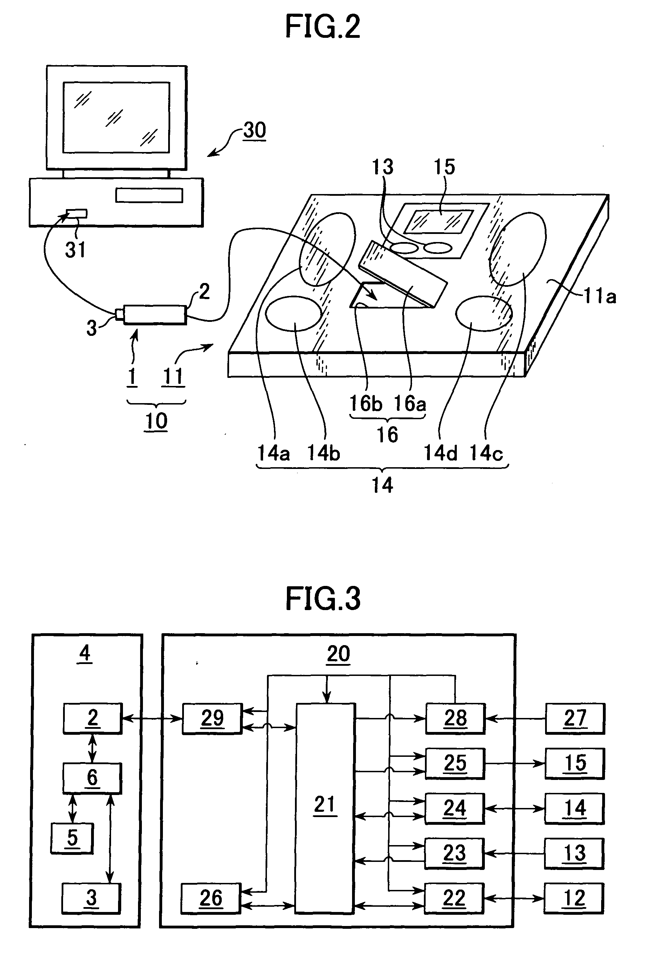

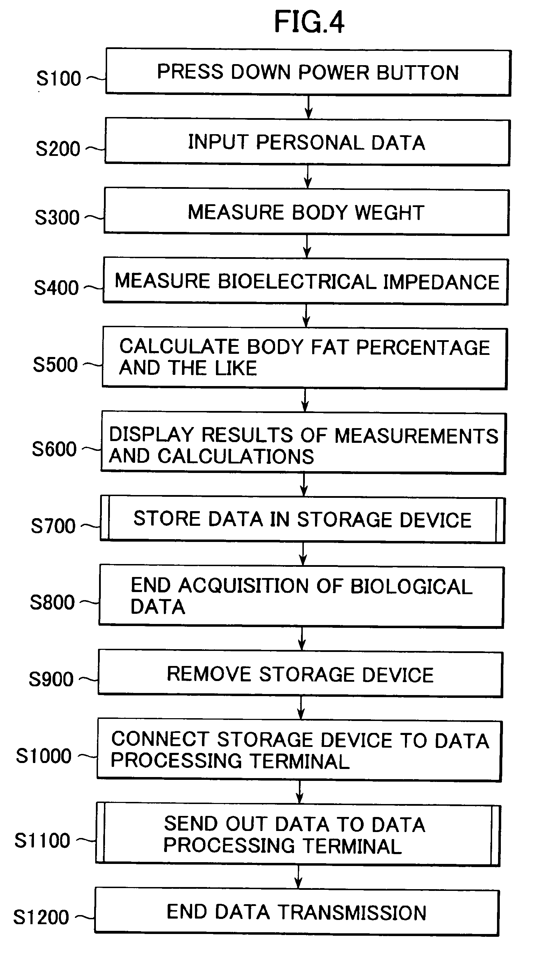

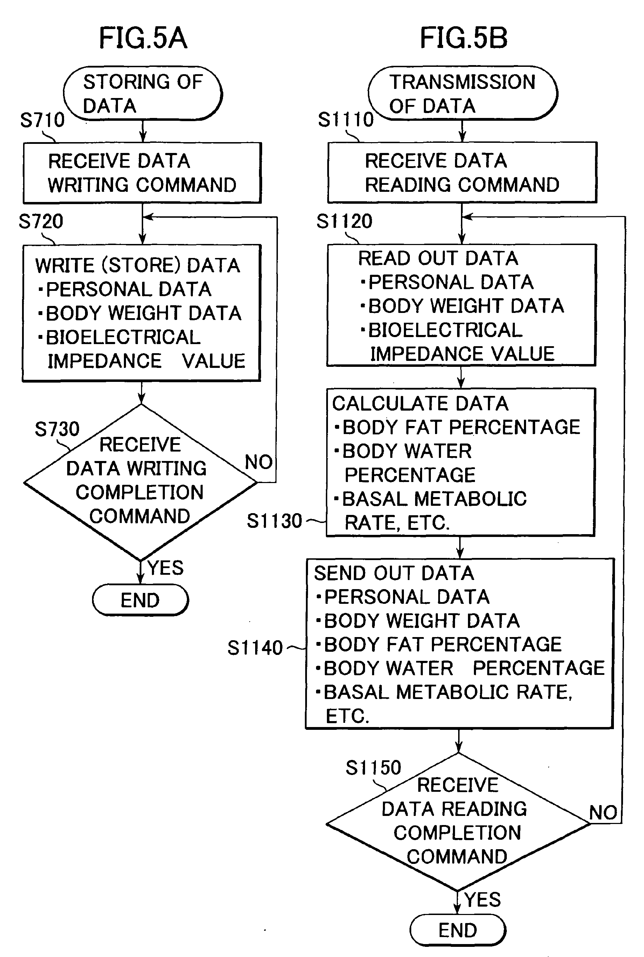

Hereinafter, a first suitable embodiment of the present invention will be described with referring to the drawings. In Fig. 1, a storage device 1 for a biological data acquiring apparatus according to the present invention is shown. In Fig. 2, together with a data processing terminal 30, a biological data acquiring apparatus 10 according to the present invention which uses the storage device 1 is shown. Further, Fig. 3 is a schematic diagram showing the constitutions of electronic circuit boards incorporated in the biological data acquiring apparatus 10. Figs. 4 and 5 are flowcharts showing steps in acquiring biological data by use of the biological data acquiring apparatus 10 and sending the acquired biological data to the data processing terminal 30.

The storage device 1 is an improved version of a so-called USB memory which has already been commercially available in various forms as an external memory for a personal computer having a terminal which meets the universal serial bus (USB) standard. The storage device 1 has a size and a shape which are nearly the same as those of a chewing gum package. As shown in Fig. 1, it has a connection terminal 2 for the main unit 11 of the biological data acquiring apparatus 10, at one end in its longitudinal direction, and has a connection terminal 3 for the data processing terminal 30, at the other end. Of the two, the connection terminal 3 is a male USB terminal and can be easily connected to a variety of data processing terminals having female USB terminals. As a matter of course, the connection terminal 3 may also be a female USB terminal or a terminal of different standard and shape from the USB standard. Alternatively, the connection terminal 2 may also be a USB terminal, or the connection terminals 2 and 3 may be combined into one connection terminal which connects the storage device 1 to the main unit 11 and the data processing terminal 30.

The main unit 11 of the biological data acquiring apparatus 10 is an improved version of a so-called scale with a body fat monitor which has already been commercially available in various forms as a stationary apparatus which calculates a body fat percentage, a body water percentage, a visceral fat level, a muscle mass, a bone density, a basal metabolic rate and the like (hereinafter generically and simply referred to as "body fat percentage" in the present embodiment) by measuring the body weight and biological impedance value of a user. As shown in Fig. 2, its top surface 11a serves as a scale platform for measuring a body weight which uses a straining member (i.e., loadcell) 12 (refer to Fig. 3). On the top surface 11a, there are provided operation switches 13 which are used by a user to enter a date and time of measurement and biological data (hereinafter generically referred to as "personal data" in the present embodiment) such as a height, age and sex, electrodes 14 which make contact with the bottoms of the feet of a user so as to measure a biological impedance value, a liquid crystal display 15 for displaying the results of measurements and calculations, and a mounting section 16 to which the storage device 1 is attached.

The electrodes 14 comprise electrodes 14a and 14b which make contact with the bottom of the left foot of a user and electrodes 14c and 14d which make contact with the bottom of the right foot of the user. Of these electrodes, a feeble electric current can be passed between both feet by use of the electrodes 14a and 14c which make contact with the toe sides of both feet, and an impedance value between both feet can be measured by use of the electrodes 14b and 14d which make contact with the heel sides of both feet. Nearly at the center of a portion sandwiched by the electrodes 14a and 14b for the left foot and the electrodes 14c and 14d for the right foot on the top surface 11a of the main unit, the mounting section 16 for the storage device 1 is formed. The mounting section 16 is formed as a cavity 16b having an openable/closable cover 16a with a sufficient size in which the storage device 1 can be set in a laid position. On the internal surface of the cavity 16b, there is provided a connection terminal 29 (refer to Fig. 3) which corresponds to the connection terminal 2 of the storage device 1.

When the mounting section 16 for the storage device 1 is formed on the top surface 11a of the main unit 11 as described above, the storage device 1 can be attached and removed more easily than when the mounting section 16 is formed on the side or rear surface of the main unit 11. Further, when the mounting section 16 has the foregoing structure that the storage device 1 can be set therein in a laid position, the storage device 1 can be prevented from sticking out from the top surface 11a at the time of its attachment without increasing the thickness of the main unit 11 so much, thereby making it difficult to occur that a user mistakenly steps on the storage device 1 and breaks it. Further, with the openable/closable cover 16a, the possibility of occurrence of such a breakage is further reduced to a significant degree. As a matter of course, the position and shape of the mounting section 16 are not limited to those in the present embodiment and may be any appropriate position and shape according to the sizes and other features of the storage device 1 and main unit 11. For example, a biological data acquiring apparatus is in existence, into which the operation switches 13 and the liquid crystal display 15 have been installed as units detachable or independent from the main unit 11. In the case of such a biological data acquiring apparatus, the mounting section 16 may be formed in the unit.

The biological data acquiring apparatus 10 (storage device 1 and main unit 11) incorporates electronic circuit boards having the constitutions shown in Fig. 3. A board 4 which is incorporated in the storage device 1 has the connection terminal 2, the connection terminal 3, a flash memory 5 as biological data storing means, and a microprocessor 6. The microprocessor 6 comprises a ROM which stores program software for writing biological data in the flash memory 5, reading biological data from the flash memory 5 and performing calculations of the biological data, a CPU and a RAM as operation elements for executing the program software, and other elements. The storage device 1 is supplied with electric power from the main unit 11 when connected to the main unit 11 or from the data processing terminal 30 when connected to the data processing terminal 30. It is needless to say that the biological data storing means may be any rewritable storage medium capable of holding data. Therefore, for example, an EEPROM can be used in place of the flash memory 5.

Meanwhile, a board 20 which is incorporated in the main unit 11 has a microprocessor 21 which comprises a CPU, a ROM and a RAM for measuring and calculating the body weight and body fat percentage of a user. To the microprocessor 21, a scale circuit 22 which is connected to the straining member 12 for measuring a body weight, an input circuit 23 which is connected to the operation switches 13 for inputting personal data, an impedance circuit 24 which is connected to the electrodes 14 for measuring a bioelectrical impedance, a display circuit 25 which is connected to the liquid crystal display 15 for displaying measured or calculated biological data such as a body weight and a body fat percentage, an EEPROM 26 which stores measured or calculated biological data, an electric power source circuit 28 which is connected to a battery 27 for driving the biological data acquiring apparatus 10, and a connection terminal 29 which corresponds to the connection terminal 2 of the storage device 1 are connected. The microprocessor 21, the straining member 12 and the scale circuit 22, the operation switches 13 and the input circuit 23, the electrodes 14 and the impedance circuit 24, and other components all together constitute biological data acquiring means incorporated in the main unit 11.

Meanwhile, the data processing terminal 30 shown in Fig. 2 is a so-called desktop personal computer in the present embodiment. It has an USB-standard-based connection terminal 31 for connecting to an external peripheral device and can be easily connected with the connection terminal 3 of the storage device 1 accordingly. In the data processing terminal 30, a program software for not only accumulating various data acquired by the biological data acquiring apparatus 10 such as personal data, body weight data and a body fat percentage continuously but also displaying histories of changes of the data as graphs on a monitor (hereinafter referred to as "history management software" in the present embodiment) is installed. As a matter of course, program software to be installed in the data processing terminal 30 is not limited to such history management software. For instance, software which gives a user an advice regarding health management based on the data acquired by the biological data acquiring apparatus 10 such as a body weight and a body fat percentage and software which sends these data to others (such as a personal doctor) via the Internet are conceivable.

Next, a flow of operations in acquiring data such as personal data, body weight data and a body fat percentage by use of the biological data acquiring apparatus 10 and sending the acquired data to the data processing terminal will be described.

In the flowchart of Fig. 4, firstly, when a user presses down a power button (not shown) on the main unit 11 (S100), an electric current from the battery 27 is fed to the circuits and the microprocessor 21 on the board 20 via the electric power source circuit 28. At this time, if the storage device 1 is set in the mounting section 16 and the connection terminal 2 is connected to the connection terminal 29, the electric current is also supplied to the storage device 1 via the connection terminals 29 and 2.

Then, the user operates the operation switches 13 so as to input personal data (S200). The personal data is sent to the microprocessor 21 via the input circuit 23 and stored in the RAM of the microprocessor 21 temporarily. It is also possible that the personal data is stored in the EEPROM 26 together with a personal number set for each user and the user causes the microprocessor 21 to retrieve the personal data from the EEPROM 26 simply by entering the personal number through the operation switches 13 on the next measurement.

Then, when the user stands on the main unit 11 with the bottoms of both feet in contact with the electrodes 14, the strain of the straining member 12 is input into the microprocessor 21 via the scale circuit 22 so as to measure a body weight (S300). At the same time, an electric current value detected by the electrodes 14 is input into the microprocessor 21 via the impedance circuit 24 so as to measure a bioelectrical impedance value (S400).

Then, the CPU in the microprocessor 21 executes program software stored in the ROM so as to calculate the body fat percentage of the user based on the personal data input in S200, the body weight data measured in S300 and the bioelectrical impedance value measured in S400 (S500). That is, this program software includes a regression formula for calculating a body fat percentage based on the personal data, the body weight data and the bioelectrical impedance value (hereinafter referred to as "regression operation software" in the present example).

Then, the microprocessor 21 displays the body weight data measured in S300 and the data such as the body fat percentage calculated in S500 on the liquid crystal display 15 via the display circuit 25 (S600). At this time, if the storage device 1 is set in the mounting section 16 and the connection terminal 2 is connected to the connection terminal 29, predetermined biological data to be described later is stored in the flash memory 5 as the biological data storing means which is incorporated in the storage device 1 (S700).

Hereinafter, the storing operation in S700 will be described in accordance with the flowchart in Fig. 5A in addition to Fig. 4. Firstly, after completion of S600 shown in Fig. 4, the microprocessor 21 of the main unit 11 sends a data writing command to the storage device 1 and also starts transmission of biological data to be described later. Upon receipt of the data writing command (S710), the microprocessor 6 of the storage device 1 writes and stores the biological data sent from the main unit 11 in the flash memory 5 sequentially (S720). The biological data sent from the main unit 11 to the storage device 1 and stored in the flash memory 5 are the personal data input in S200, the body weight data measured in S300 and the bioelectrical impedance value measured in S400. The data of the body fat percentage calculated in S500 is however not stored in the flash memory 5 so as to reduce the capacity of the flash memory 5.

Then, when a data writing completion command is sent from the microprocessor 21 of the main unit 11 to the microprocessor 6 of the storage device 1 (S730) after completion of writing of the biological data to the flash memory 5, the operation of storing the biological data into the flash memory 5 in S700 of Fig. 4 is completed. Thereby, the operation of acquiring the personal data, the body weight data and the data of the body fat percentage by use of the biological data acquiring apparatus 10 is completed (S800).

Then, in order to transfer the personal data, the body weight data and the data of the body fat percentage acquired by the biological data acquiring apparatus 10 to the data processing terminal 30, the user removes, from the main unit 11, the storage device 1 which is independent and detachable from the main unit 11 (S900), carries the storage device 1 to where the data processing terminal 30 is placed, and connects the connection terminal 3 to the connection terminal 31 of the data processing terminal 30 (S1000). Then, for example, when the history management software installed in the data processing terminal 30 is activated, the personal data, the body weight data and the data of the body fat percentage are sent out from the storage device 1 to the data processing terminal 30 (S1100).

Hereinafter, the data sending operation in S1100 will be described in accordance with the flowchart of Fig. 5B in addition to Fig. 4. Firstly, in accordance with the history management software, the data processing terminal 30 sends a data reading command to the storage device 1. Upon receipt of the data reading command (S1110), the microprocessor 6 of the storage device 1 reads out the personal data, body weight data and bioelectrical impedance value stored in the flash memory 5 (S1120) and executes program software stored in the ROM so as to calculate the body fat percentage (S1130). The program software executed above is the same as the regression operation software stored in the microprocessor 21 of the main unit 11. Then, the personal data, the body weight data and the data of the body fat percentage are sent from the connection terminal 3 to the data processing terminal 30 (S1140).

Thereafter, when a data reading completion command is sent from the data processing terminal 30 to the microprocessor 6 of the storage device 1 (S1150) after completion of receipt of the personal data, the body weight data and the data of the body fat percentage in the data processing terminal 30, the data sending operation in S1100 of Fig. 4 is completed. Thereby, the operation of sending out the personal data, the body weight data and the data of the body fat percentage by use of the storage device 1 of the biological data acquiring apparatus 10 is completed (S1200).

As described above, the storage device 1 of the biological data acquiring apparatus 10 in the present embodiment is independent and detachable from the main unit 11 and has the flash memory 5 for storing the personal data, body weight data and bioelectrical impedance value which are acquired by means of the straining member 12, the operation switches 13, the electrodes 14 and the microprocessor 21, those being incorporated in the main unit 11. Thus, the storage device 1 can be removed from the main unit 11 with the personal data, body weight data and bioelectrical impedance value stored in the flash memory 5. Further, the storage device 1 can be connected to the data processing terminal 30 so as to send the personal data and body weight data stored in the flash memory 5 to the data processing terminal 30.

In other words, the biological data acquiring apparatus 10 in the present embodiment comprises the main unit 11 and the storage device 1; the main unit incorporating the straining member 12, the operation switches 13, the electrodes 14 and the microprocessor 21, for acquiring the personal data, the body weight data and the data of the body fat percentage; while, the storage device 1 being independent and detachable from the main unit 11 and having the flash memory 5 for storing the personal data, body weight data and bioelectrical impedance value which are acquired by the main unit 11. The biological data acquiring apparatus 10 therefore enables the storage device 1 to be removed from the main unit 11 with the personal data, body weight data and bioelectrical impedance value stored in the flash memory 5. Further, the storage device 1 can be connected to the data processing terminal 30 so as to send the personal data and body weight data stored in the flash memory 5 to the data processing terminal 30.

Further, the storage device 1 has the microprocessor 6 which comprises the ROM containing the regression operation software for calculating the body fat percentage based on the personal data, body weight data and bioelectrical impedance value stored in the flash memory 5, and the CPU and RAM for executing the regression operation software. Thus, calculation of the body fat percentage can be performed in the storage device 1, and then the data of the calculated body fat percentage can be sent to the data processing terminal 30. As a result, there is no need to store all the data of the body fat percentage in the flash memory 5, and a flash memory 5 with a small capacity is used to make the storage device 1 or the biological data acquiring apparatus 10 inexpensive.

Further, although the main unit 11 is an improved version of a stationary scale with a body fat monitor, there is no need to move the main unit 11 when the personal data, the body weight data and the data of the body fat percentage are sent to the data processing terminal 30, thereby making the biological data acquiring apparatus 10 easy to use.

In the present embodiment, the microprocessor 6 which stores and further executes the regression operation software is included in the storage device 1. However, it is also possible that the storage device 1 merely stores such regression operation software and the data processing terminal 30 actually executes the software. In this case, the storage device 1 is connected to the data processing terminal 30 so as to send the personal data, body weight data and bioelectrical impedance value thereto and subsequently the data processing terminal 30 accesses and executes the regression operation software stored in the storage device 1 by utilizing the data and the value, so as to receive the data of the calculated body fat percentage from the storage device 1. With such a constitution as well, there is no need to store all the data of the body fat percentage in the flash memory 5, so that a flash memory 5 with a small capacity can be used to make the storage device 1 and the biological data acquiring apparatus 10 inexpensive.

It is also possible that the regression operation software is executed only by the microprocessor 21 of the main unit 11 and the data of the calculated body fat percentage is also stored in the flash memory 5 of the storage device 1. In this case as well, it is possible to send the personal data, the body weight data and the data of the body fat percentage to the data processing terminal 30 by use of the storage device 1.

In addition, the constitutions of the storage device and biological data acquiring apparatus to which the present invention is applied do not have to be limited to descriptions in the present embodiment, and various modifications can be made on the constitutions, as has been described accordingly together with the description of the embodiment. Further, the present invention is not limited to an apparatus which measures bioelectrical impedance between the bottoms of both feet of a user as in the embodiment and can also be widely applied to apparatuses which measure bioelectrical impedance between both hands, between a hand and a foot or between other specific body parts of a user. Further, the present invention is not limited to a biological data acquiring apparatus of the type which measures the body weight or bioelectrical impedance of a user as in the present embodiment and can also be widely applied, for example, to an apparatus which measures or calculates a thickness of fat or a bone density by use of the ultrasonic wave or the like as well as to various known apparatuses for acquiring biological data, e.g., a blood pressure meter, a pulse meter, a pedometer and a consumed calorie meter. Further, it is needless to say that the present invention can be widely applied to portable biological data acquiring apparatuses, in addition to stationary biological data acquiring apparatuses.

### [Example 2]

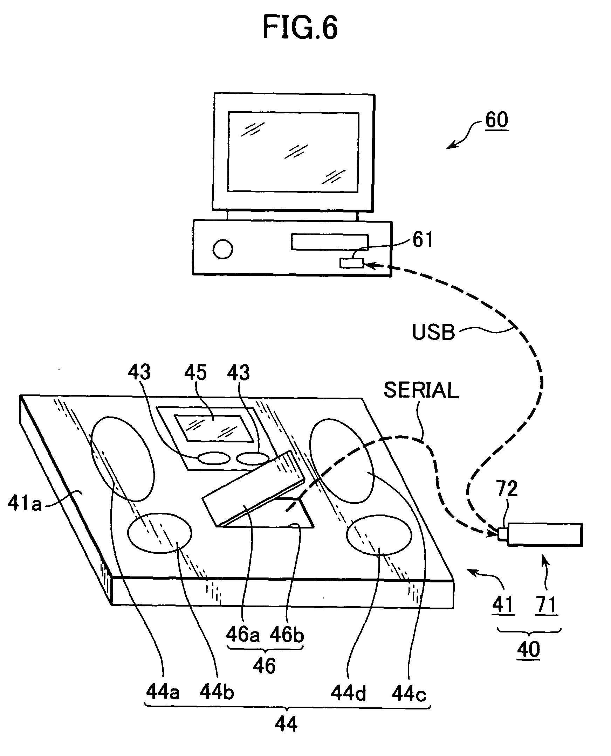

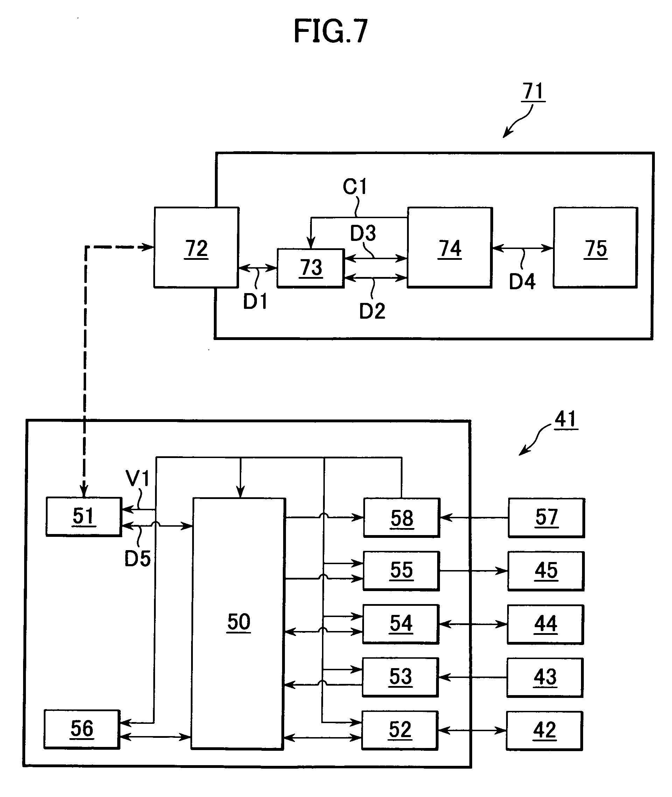

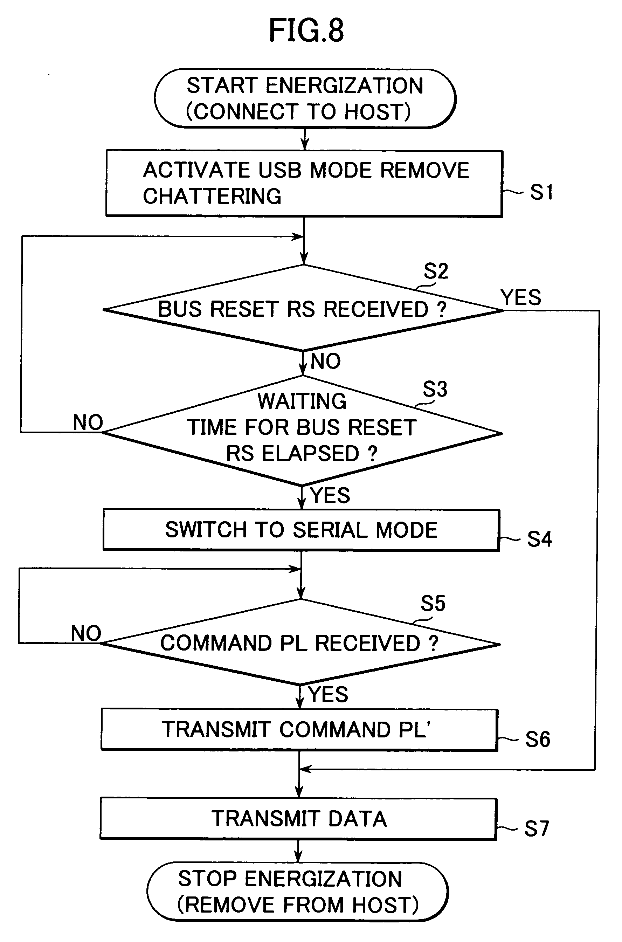

Hereinafter, a second embodiment of the present invention will be described with referring to the drawings. Fig. 6 shows an external view of a biological data acquiring apparatus 40 as a suitable embodiment of the present invention together with a data processing terminal 60. Fig. 7 is a schematic diagram showing the circuit configuration of the biological data acquiring apparatus 40. Figs. 8 to 13 are flowcharts showing a control program which is executed in the biological data acquiring apparatus 40 and diagrams showing signals on data buses at the time of execution of the control program.

As shown in Figs. 6 and 7, the biological data acquiring apparatus 40 comprises a main unit 41 and a storage device 71 which is detachable from the main unit 41. The biological data acquiring apparatus 40 can send biological data acquired in the main unit 41, such as a body fat percentage (mass) , a body water percentage (level), a visceral fat level, a muscle mass (percentage), a leg muscle mass (percentage), a bone mass, a bone density and a basal metabolic rate (hereinafter generically and simply referred to as "body fat percentage" in the present embodiment) , to the data processing terminal 60 by use of the storage device 71 as indicated by the dotted lines.

The storage device 71 is an improved version of a conventional USB memory which has already been commercially available in various forms as an external memory for a personal computer having a terminal which meets the USB standard. The storage device 71 has a size and a shape which are nearly the same as those of a chewing gum package. It has a male USB terminal 72 at one end in its longitudinal direction and can be easily connected, via the USB terminal 72, to the main unit 41 and the data processing terminal 60 which have a female USB terminal 61. The storage device 1 carries data between the main unit 41 and the data processing terminal 60 which serve as host devices.

Further, the main unit 41 is an improved version of a so-called scale with a body fat monitor which has already been commercially available in various forms as a stationary apparatus which calculates the body fat percentage by measuring the body weight and bioelectrical impedance value of a user. Its top surface 41a serves as a scale platform for measuring a body weight which uses a straining member 42 (refer to Fig. 7). On the top surface 41a, there are provided operation switches 43 which are used by a user to enter a date and time of measurement and biological data such as a height, age and sex (hereinafter generically referred to as "personal data" in the present example) , electrodes 44 which make contact with the bottoms of the feet of a user so as to measure a bioelectrical impedance value, a liquid crystal display 45 for displaying the results of measurements and calculations, and a mounting section 46 to which the storage device 71 is attached.

The electrodes 44 comprise electrodes 44a and 44b which make contact with the bottom of the left foot of a user and electrodes 44c and 44d which make contact with the bottom of the right foot of the user. Of these electrodes, a feeble electric current is passed between both feet by use of the electrodes 44a and 44c which make contact with the toe sides of both feet, a potential difference (voltage) between both feet is measured by use of the electrodes 44b and 44d which make contact with the heel sides of both feet, and bioelectrical impedance can be determined from these electric current value and voltage value.

Nearly at the center of a portion sandwiched by the electrodes 44a and 44b for the left foot and the electrodes 44c and 44d for the right foot on the top surface 41a of the main unit 41, the mounting section 46 for the storage device 71 is formed. The mounting section 46 is is formed as a cavity 46b having an openable/closable cover 46a with a sufficient size in which the storage device 71 can be set in a laid position. On the internal surface of the cavity 46b, there is provided a female USB terminal 51 (refer to Fig. 7) which corresponds to the USB terminal 72 of the storage device 71.

When the mounting section 46 for the storage device 71 is formed on the top surface 41a of the main unit 41 as described above, the storage device 71 can be attached and removed more easily than when the mounting section 46 is formed on the side or rear surface of the main unit 41. Further, when the mounting section 46 has the foregoing structure that the storage device 71 can be set therein in a laid position, the storage device 71 can be prevented from sticking out from the top surface 41a at the time of its attachment without increasing the thickness of the main unit 41 so much, thereby making it difficult to occur that a user mistakenly steps on the storage device 71 and breaks it. Further, with the openable/closable cover 46a, the possibility of occurrence of such a breakage is further reduced to a significant degree.

As a matter of course, the position and shape of the mounting section 46 are not limited to those in the present embodiment and may be any appropriate position and shape according to the sizes and other features of the storage device 71 and main unit 41. For example, a biological data acquiring apparatus is in existence, into which the operation switches 13 and the liquid crystal display 45 have been installed as units detachable or independent from the main unit 41. In the case of such a biological data acquiring apparatus, the mounting section 46 may be formed in the unit.

The biological data acquiring apparatus 40 (storage device 71 and main unit 41) has a circuit configuration as shown in Fig. 7 therein.

The storage device 71 has the USB terminal 72, an analog switch 73 which is used to select a data signal to be transmitted between the storage device 71 and the main unit 41 or data processing terminal 60 which is a host device, a microprocessor 74 for implementing control associated with processing or storing of the data signal, and an EEPROM 75 for storing data. As a matter of course, the EEPROM 75 may be any rewritable storage medium capable of holding data such as a nonvolatile memory, and a flash memory may be used, for example.

The USB terminal 72 is connected to the analog switch 73 via a data bus D1. The analog switch 73 is connected to the microprocessor 74 via a data bus D2 which is used for USB transmission, a data bus D3 which is used for serial transmission and a control signal bus C1 for transmitting a signal for switching between the data buses D2 and D3. The microprocessor 74 is connected to the EEPROM 75 via a data bus D4. Further, although not shown, the analog switch 73, the microprocessor 74 and the EEPROM 75 are connected to the USB terminal 72 via an electric power supply line. Hence, the storage device 71 operates by receiving electric power from the host device via the USB terminal 72, as in the case of many conventionally known devices which meet the USB standard such as the above conventional USB memory.

In Fig. 7, the data buses D1 to D4 are indicated by bidirectional arrows for brevity. In reality, the data buses D1 to D4 are each constituted by an up data line D+ and a down data line D-. Further, in the present embodiment in particular, the data line D+ of the data bus D2 is connected to the ground side, the data line D- of the data bus D2 is connected to the electric power supply side (electric power supply line), and the data lines D+ and D- of the data bus D3 are connected to the ground side. Thereby, the data bus D2 is used for USB transmission, and the data bus D3 is used for serial transmission. Further, the control signal bus C1 is constituted by an appropriate number of signal lines corresponding to the number of connection ports of the analog switch 73.

Further, the microprocessor 74 processes a data signal conforming to the USB standard (hereinafter simply referred to as "USB signal") and a data signal conforming to a standard preset between the storage device 71 and the main unit 41 (hereinafter simply referred to as "serial signal"). Further, the microprocessor 74 executes a control program which will be described later with referring to Figs. 8 to 11 so as to output a control signal to the analog switch 73 via the control signal bus C1, thereby connecting the data bus D1 selectively to the data bus D2 or D3. That is, the microprocessor 74 constitutes data signal processing means, and the microprocessor 74 and the analog switch 73 constitute data signal selecting means. These data signal processing means and data signal selecting means and the above USB terminal constitute a data transmitter.

Meanwhile, the main unit 41 has a microprocessor 50 which comprises a CPU, a ROM and a RAM, for measuring or calculating the body weight and body fat percentage of a user. To the microprocessor 50, the USB terminal 51 which corresponds to the USB terminal 72 of the storage device 71 is connected via a data bus D5. In addition to the USB terminal 51, a scale circuit 52 which is connected to the above straining member 42, an input circuit 53 which is connected to the above operation switches 43, an impedance circuit 54 which is connected to the above electrodes 44, a display circuit 55 which is connected to the above liquid crystal display 45, an EEPROM 56 which stores biological data such as input personal data and a calculated body fat percentage, and an electric power source circuit 58 which is connected to a battery 57 for driving the biological data acquiring apparatus 40 are also connected to the microprocessor 50 via respective data buses or control signal buses.

Further, the USB terminal 51 is connected to the electric power source circuit 58 via an electric power supply line V1. The microprocessor 50, the EEPROM 56 and the above circuits are also connected to the electric power source circuit 58 via respective electric power supply lines. The electric power source circuit 58 keeps the electric power supply line V1 and the battery 57 disconnected from each other under normal circumstances and connects one to the other upon receipt of a control signal from the microprocessor 50.

Further, the microprocessor 50 calculates a body fat percentage based on a body weight measured by the straining member 42 and the scale circuit 52, personal data input by the operation switches 43 and the input circuit 53, and bioelectrical impedance measured by the electrodes 44 and the impedance circuit 54. That is, the microprocessor 50, the straining member 42 and the scale circuit 52, the operation switches 43 and the input circuit 53, and the electrodes 44 and the impedance circuit 54 all together constitute biological data acquiring means incorporated in the main unit 41.

The processing capacity of the CPU constituting the microprocessor 50 is not as high as that of a CPU incorporated in a personal computer or the like. Therefore, the CPU does not accommodate USB transmission and relies on the above serial transmission using the serial signals in transmitting data to the storage device 71.

Meanwhile, the data processing terminal 60 shown in Fig. 6 is a so-called desktop personal computer which accommodates USB transmission. It has a female USB terminal 61. Further, history management program software for displaying histories of changes of biological data as graphs on a monitor is also installed in the data processing terminal 60. By the program software, the personal data, the body weight data and the data of the body fat percentage which are acquired in the main unit 41 of the biological data acquiring apparatus 40 are USB-transmitted from the storage device 71 to the data processing terminal 60, whereby histories of changes of these biological data can be managed.

In addition to or instead of the above history management software, software which gives a user an advice regarding health management based on the above data such as a body weight and a body fat percentage and software which sends these data to others (such as a personal doctor) via the Internet are also conceivable as program software to be installed in the data processing terminal 60.

When biological data are acquired with the storage device 71 of the biological data acquiring apparatus 40 connected to the main unit 41, the acquired biological data are serial-transmitted from the main unit 41 to the storage device 71 and stored in the EEPROM 75 (as indicated by the dotted line SERIAL in Fig. 6). Further, when the storage device 71 is connected to the data processing terminal 60, the biological data stored in the EEPROM 75 are USB-transmitted to the data processing terminal 60 (as indicated by the dotted line USB in Fig. 6).

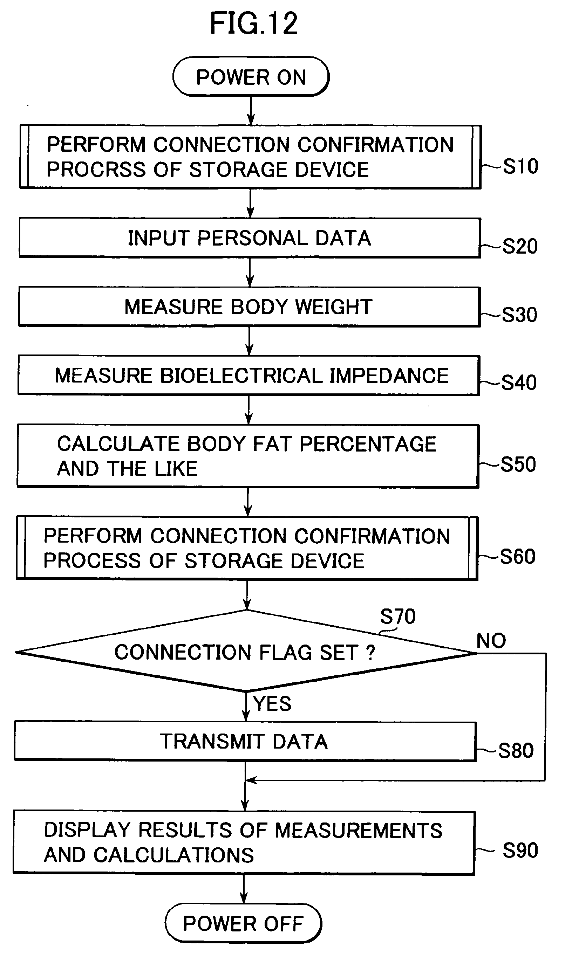

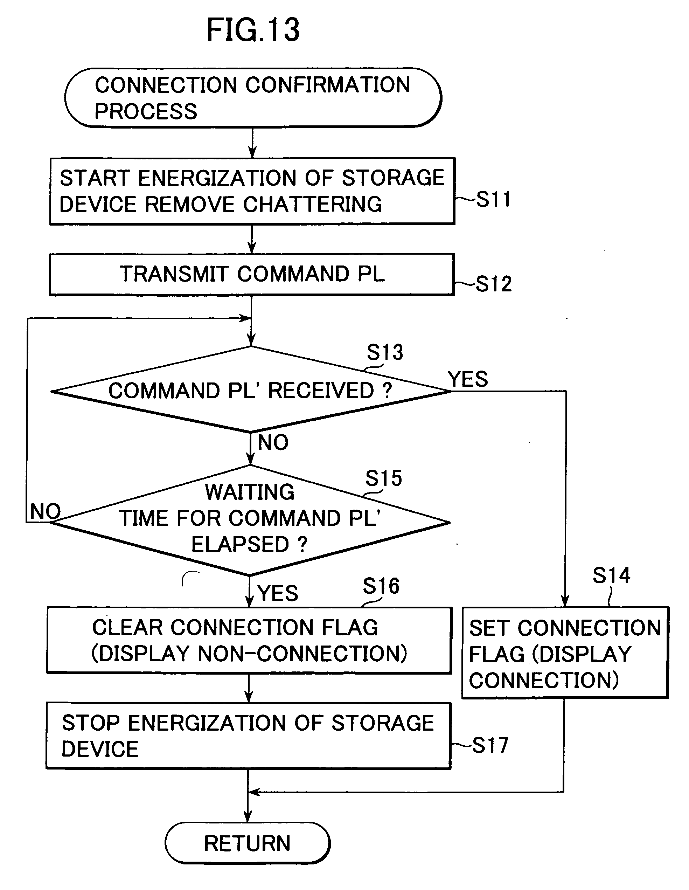

Next, the control program for switching between serial transmission and USB transmission in the storage device 71 will be described with referring to Figs. 8 to 11, and the control process in transmitting biological data from the main unit 41 to the storage device 71 will be then described with referring to Figs. 12 and 13. The description of transmission of data from the storage device 71 to the data processing terminal 60 will be omitted because the data transmission is based on known USB transmission.

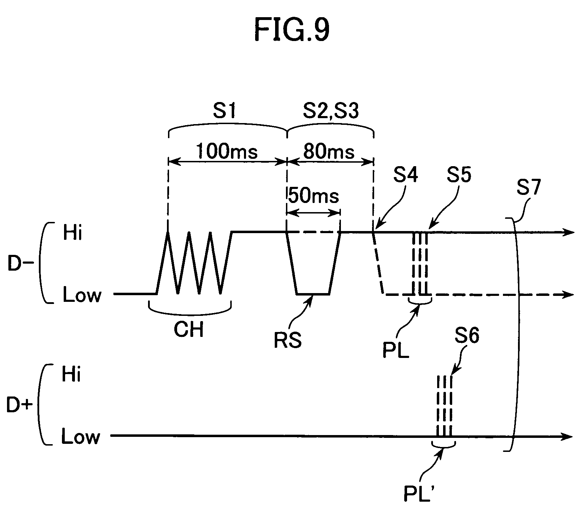

Fig. 8 is a flowchart showing a first example of a control program (hereinafter referred to as "first program") which is executed by the microprocessor 74 of the storage device 71 when the storage device 71 is connected to the main unit 41 or the data processing terminal 60 which is a host device. Further, Fig. 9 is a diagram showing a signal in the data bus D1 at the time of execution of the control program in Fig. 8. When the storage device 71 is connected to the main unit 41, the data bus D5 of the main unit 41 also shows a signal similar to that of Fig. 9.

The first program is executed in the microprocessor 74 at the start of energization of the storage device 1. That is, when a connection confirmation process to be described later is conducted in the main body 41 to which the storage device 71 is being connected, when the storage device 71 is connected to the data processing terminal 60 whose power is on or when the data processing terminal 60 is turned on with the storage device 71 being connected to the terminal 60, electric power is supplied to the storage device 71 from the main unit 41 or the data processing terminal 60 via the USB terminal 72, and control in accordance with the flowchart of Fig. 8. is implemented.

Firstly, in STEP S1, the microprocessor 74 selects and activates a USB transmission mode, regardless of whether the storage device 71 is being connected to the main unit 41 or the data processing terminal 60. That is, the microprocessor 74 transmits a switching signal to the analog switch 73 via the control signal bus C1 so as to connect the data bus D1 to the data bus D2 which accommodates USB transmission. As a result, the data buses D1 and D2 are connected to each other, and the data line D- of the data bus D1 is pulled up to the Hi level as indicated by a solid line in Fig. 9, and USB transmission becomes possible.

Further, in STEP S1, a waiting time of 100 ms is set so as to remove chattering CH at the moment when the USB terminal 72 is connected to the USB terminal of the host device. The waiting time may be any duration of time as long as it is sufficient to remove the chattering. The waiting time is not limited to 100 ms and may be preset to time of given length. After the waiting time of 100 ms elapses, the microprocessor 74 proceeds to STEP S2.

In STEP S2, reception of a bus reset signal RS is confirmed. The bus reset signal RS is an identification signal for confirming connection which is transmitted from the host device upon connection in the USB standard. That is, when the storage device 71 is connected to the data processing terminal 60, the bus reset signal RS is received, and the data line D- becomes the Low level over 50 ms (or 10 ms when a USB hub intervenes) (as indicated by a solid line in Fig. 9). When the reception of the bus reset signal RS is confirmed, the microprocessor 74 determines that the storage device 71 is connected to the data processing terminal 60 and proceeds to STEP S7 by skipping subsequent STEPS S3 to S6. Meanwhile, when the reception of the bus reset signal RS is not confirmed, the microprocessor 74 proceeds to STEP S3.

In STEP S3, it is determined whether time to wait for the reception of the bus reset signal RS has been elapsed. Timing of transmission of the bus reset signal RS from the host device becomes slightly earlier or later than it should be depending on the loaded condition of the device or other conditions. Thus, in the present example, a waiting time of 80 ms is set. As a matter of course, the waiting time may be set as appropriate. When the waiting time of 80 ms has not yet been elapsed, the microprocessor 74 returns to STEP S2 and confirms the reception of the bus reset signal RS again. Thus, STEP S2 and STEP S3 are repeated until the bus reset signal RS is confirmed in STEP S2 or passage of the waiting time of 80 ms is confirmed in STEP S3. Meanwhile, when it has been confirmed in STEP S3 that the waiting time of 80 ms has been elapsed, the microprocessor 74 proceeds to STEP S4.

In STEP S4, the USB transmission mode is switched to a serial transmission mode. That is, when the bus reset signal RS is not received within the waiting time in STEP S3, the microprocessor 74 determines that the storage device 71 is connected to the main unit 41 (that is, a device which does not accommodate USB transmission) and transmits a switching signal to the analog switch 73 via the control signal bus C1 so as to connect the data bus D1 to the data bus D3 which accommodates serial transmission. As a result, the data buses D1 and D3 are connected to each other, and the data line D- is pulled down to the Low level as indicated by a dotted line in Fig. 9, and serial transmission becomes possible. Thereafter, the microprocessor 74 proceeds to STEP S5.

In STEP S5, reception of a command pulse PL is confirmed. The command pulse PL is an identification signal for confirming connection which is preset to be transmitted from the main unit 41 to the storage device 71 in executing a connection confirmation process to be described later. Therefore, when the reception of the command pulse PL has been confirmed, the microprocessor 74 determines that the storage device 71 is connected to the main unit 41 and proceeds to STEP S6. Meanwhile, when the command pulse PL is not confirmed, it implies that the storage device 71 is in such an abnormal state that its power is on without being connected to either the main unit 41 or the data processing terminal 60 (for example, contact failure of the data bus for the USB terminal 72). Hence, the microprocessor 74 repeats STEP S5 until the command pulse PS is confirmed.

In STEP S6, the microprocessor 74 transmits a reply command pulse PL' which is preset in conformity to the command pulse PL to the main unit 41. Thereby, the microprocessor 50 of the main unit 41 also confirms connection of the storage device 71. Then, the microprocessor 74 proceeds to STEP S7.

In STEP S7, data is transmitted between the storage device 71 and the main unit 41 or the data processing terminal 60 in accordance with the transmission mode selected by STEPS S1 to S4. That is, when the storage device 71 is connected to the main unit 41 and the serial transmission mode is selected, the microprocessor 74 receives serial signals from the main unit 41 as biological data via the USB terminal 72, the data bus D1, the analog switch 73 and the data bus D3 and stores the data in the EEPROM 75 via the data bus D4. Meanwhile, when the storage device 71 is connected to the data processing terminal 60 and the USB transmission mode is selected, the microprocessor 74 reads out biological data from the EEPROM 75 via the data bus D4 and transmits to the data processing terminal 60 as the USB signals via the data bus D2, the analog switch 73, the data bus D1 and the USB terminal 72.

Thereafter, by removing the storage device 71 from the main unit 41 or the data processing terminal 60 or by turning off the main unit 41 or the data processing terminal 60, the supply of electric current to the storage device 71 is also shut off, and the whole control process is completed.

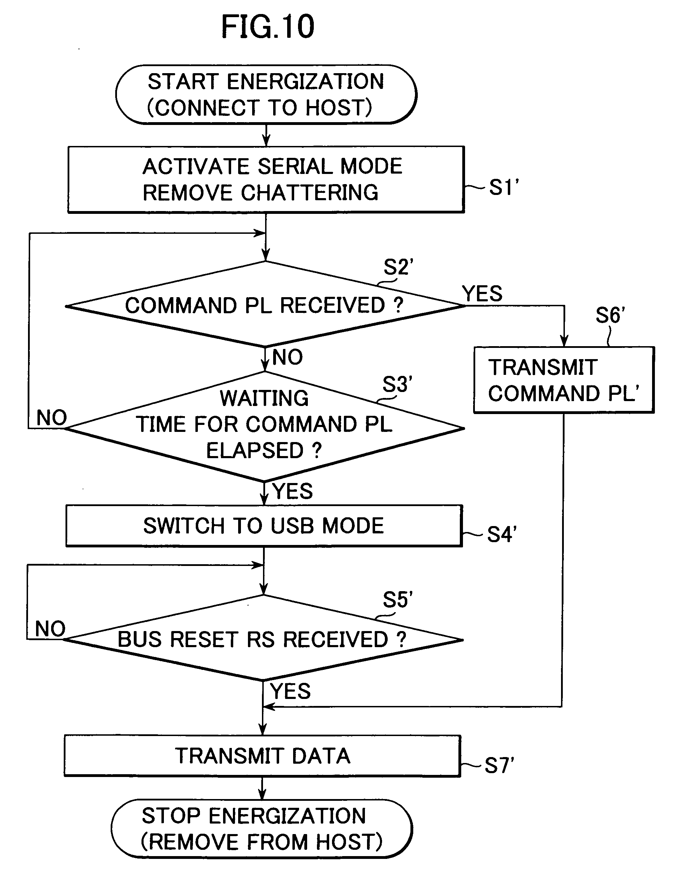

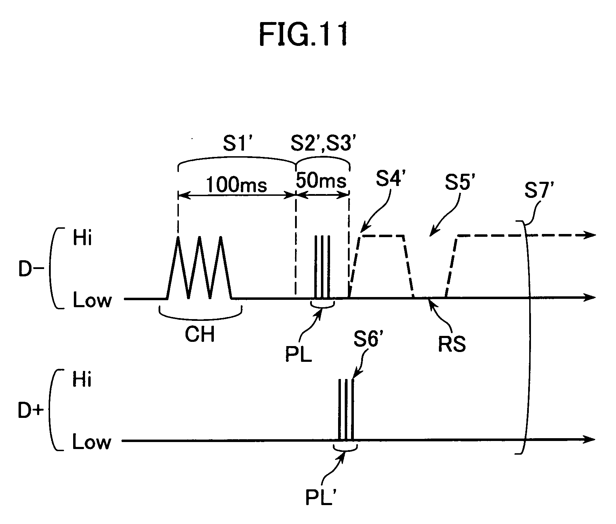

Fig. 10 is a flowchart showing a second example of the control program (hereinafter referred to as "second program") which is executed by the microprocessor 74 of the storage device 71 when the storage device 71 is connected to the host device. Further, Fig. 11 is a diagram showing a signal in the data bus D1 at the time of execution of the control program in Fig. 10.

In this second program, firstly, the microprocessor 74 selects and activates a serial transmission mode in STEP S1' after the start of energization. That is, the data buses D1 and D3 are connected to each other via the analog switch 73, the data line D- becomes the Low level as indicated by a solid line in Fig. 11, and serial transmission becomes possible. Further, in STEP S1' as well, a waiting time of 100 ms is set so as to remove chattering CH.

Then, in STEP S2', reception of the command pulse PL is confirmed. When the command pulse PL is confirmed, the microprocessor 74 can determine that the storage device 71 is connected to the main unit 41 and therefore proceeds to STEP S6'. In STEP S6', the microprocessor 74 transmits the reply command pulse PL' to the main unit 41 and then proceeds to STEP S7'. Meanwhile, when the command pulse PL is not confirmed in STEP S2', the microprocessor 74 proceeds to STEP S3'.

In STEP S3', it is determined whether time to wait for the reception of the command pulse PL has been elapsed. In the present example, a waiting time of 50 ms is set. When the waiting time of 50 ms has not been elapsed, the microprocessor 74 returns to STEP S2' and confirms the reception of the command pulse PL again. Thus, STEP S2' and STEP S3' are repeated until the command pulse PL is confirmed in STEP S2' or passage of the waiting time of 50 ms is confirmed in STEP S3'. Meanwhile, when it has been confirmed in STEP S3' that the waiting time of 50 ms has already been elapsed, the microprocessor 74 proceeds to STEP S4'. Since the command pulse PL is transmitted from not the data processing terminal 60 such as a personal computer which has a high load change rate but the main unit 41 which is a device with a relatively stable load, timing of its transmission can be relatively stable as compared with timing of transmission of the bus reset signal RS.

In STEP S4', the serial transmission mode is switched to a USB transmission mode. That is, when the command pulse PL is not received within the waiting time in STEP S3', the microprocessor 74 determines that the storage device 71 is not connected to the main unit 41 and transmits a control signal to the analog switch 73 so as to connect the data bus D1 to the data bus D2 which is used for USB transmission. As a result, the data line D- of the data bus D1 is pulled up to the Hi level as indicated by a dotted line in Fig. 11, and serial transmission becomes possible. Thereafter, the microprocessor 74 proceeds to STEP S5'.

In STEP S5', reception of the bus reset signal RS is confirmed. When the bus reset signal RS is confirmed, the microprocessor 74 can determine that the storage device 71 is connected to the data processing terminal 60 and then proceeds to STEP S7'. Meanwhile, when the bus reset signal RS is not confirmed, it implies that the storage device 71 is in such an abnormal state that its power is on without being connected to either the main unit 41 or the data processing terminal 60 (for example, contact failure of the data bus for the USB terminal 72). Thus, the microprocessor 74 repeats STEP S5' until the bus reset signal RS is confirmed.

In STEP S7', data is transmitted between the storage device 71 and the main unit 41 or the data processing terminal 60 in accordance with the transmission mode selected by STEPS S1' to S4'. A detailed description of the data transmission will be omitted because STEP S7' is similar to STEP S7 of the first program.

Thereafter, by removing the storage device 71 from the main unit 41 or the data processing terminal 60 or by turning off the main unit 41 or the data processing terminal 60, the supply of electric current to the storage device 71 is also shut off, and the whole control process is completed.

A difference between the first program and the second program will be described. In the first program, when the data processing terminal 60 is under an extremely large load and consequently the timing of transmission of the bus reset signal RS is significantly delayed, the storage device 71 may misrecognize that it is connected to the main unit 41. On the other hand, in the second program, although it must be preset to transmit a given command pulse PL from the main unit 41, the timing of transmission of the pulse is stable, so that the possibility of occurrence of the above misrecognition can be reduced.

Next, the control process in acquiring biological data such as personal data, body weight data and a body fat percentage by the main unit 41 of the biological data acquiring apparatus 40 and transmitting the biological data to the storage device 71 will be described with referring to Figs. 12 and 13.

Fig. 12 shows the main routine of a control program which is executed by the microprocessor 50 of the main unit 41. When a user presses down a power button (not shown) on the main unit 41 so as to turn on the main unit 41, this control program is executed after a predetermined initialization process.

Firstly, in STEP S10, the process of confirming connection of the storage device 71 is performed. In this process, it is confirmed whether the storage device 71 is connected to the main unit 41 correctly and data can be transmitted from the main unit 41 to the storage device 71, and if the data transmission is possible, a connection flag is set, while if the data transmission is not possible, the connection flag is cleared. This connection confirmation process will be further described later.

Then, in STEP S20, the user operates the operation switches 43 so as to input personal data. The personal data is sent to the microprocessor 50 via the input circuit 53 and stored in the RAM of the microprocessor 50 temporarily. It is also possible that the personal data is stored in the EEPROM 56 together with a personal number set for each user and the user causes the microprocessor 50 to retrieve the personal data from the EEPROM 56 simply by entering the personal number through the operation switches 43 on the next measurement.

Then, in STEP S30, when the user stands on the main unit 41 with the bottoms of both feet in contact with the electrodes 44, the strain of the straining member 42 is input into the microprocessor 50 via the scale circuit 52 so as to measure a body weight. Further, in STEP S40, a voltage value detected by the electrodes 44 is input into the microprocessor 50 via the impedance circuit 54 so as to measure bioelectrical impedance. In addition, in STEP S50, the body fat percentage of the user are calculated based on the personal data input in STEP S20, the body weight data measured in STEP S30 and the bioelectrical impedance measured in STEP S40. The processes of STEPS S20 to S50 are similar to those carried out by a conventionally known scale with a body fat monitor.

Then, in STEP S60, the process of confirming connection of the storage device 71 is performed again. This process is the same as the process in STEP S10.

Then, in STEP S70, the state of the connection flag which has been set or cleared in STEP S10 and STEP S60 is determined. When the connection flag is set, the microprocessor 50 proceeds to STEP S80, while when the connection flag is cleared, the microprocessor 50 skips STEP S80 and proceeds to STEP S90.

In STEP S80, the biological data acquired in STEPS S20 to S50 are serial-transmitted to the storage device 71.

Then, in STEP S90, the biological data acquired in STEPS S20 to S50 are displayed on the liquid crystal display 45. This display process is also similar to that carried out by the conventionally known scale with a body fat monitor. Thereafter, the main unit 41 is turned off, whereby the whole control process is completed.

Next, the connection confirmation process which is carried out in STEP S10 and STEP S60 will be described with referring to the flowchart in Fig. 13 which shows the subroutine of the connection confirmation process. Firstly, in STEP S11, the microprocessor 50 controls the electric power source circuit 58 so as to connect the electric power supply line V1 to the battery 57. As a result, when the storage device 71 is connected to the main unit 41, the electric power of the battery 57 is supplied from the electric power source circuit 58 to the storage device 71 via the electric power supply line V1, the USB terminal 51 and the USB terminal 72. Thus, energization of the storage device 71 is initiated.

Further, in STEP S11, a predetermined waiting time is set so as to remove the above chattering CH. This waiting time desirably corresponds to the waiting time set for the storage device 71. That is, when the storage device 71 uses the above first program, the predetermined waiting time is desirably at least the sum of 80 ms and 100 ms (i.e. 180 ms), which are respectively set in STEP S3 and S1 of the first program, and is set at 200 ms, for example. Meanwhile, when the storage device 71 uses the above second program, the predetermined waiting time is desirably not shorter than the waiting time of 100 ms which is set in STEP S1' of the second program and is set at 120 ms, for example. After the thus set waiting time is elapsed, the microprocessor 50 proceeds to STEP S12.

Then, in STEP S12, the microprocessor 50 transmits the command pulse PL for confirming connection to the USB terminal 51 via the data bus D5. When the storage device 71 is connected to the main unit 41, the command pulse PL from the USB terminal 51 is received by the microprocessor 74 of the storage device 71 via the USB terminal 72.

Then, in STEP S13, the microprocessor 50 confirms reception of the reply command pulse PL' from the storage device 71. The reply command pulse PL' is set to be transmitted by the storage device 71 when the storage device 71 receives the command pulse PL (STEP S6 in Fig. 8 or STEP S6' in Fig. 10). Thus, when the reply command pulse PL' is confirmed, the microprocessor 50 determines that the storage device 71 is connected to the main unit 41 and proceeds to STEP S14. Meanwhile, when the reply command pulse PL' is not confirmed, the microprocessor 50 proceeds to STEP S15.

In STEP S14, the microprocessor 50 sets the connection flag and displays a mark or the like which indicates connection of the storage device 71 on the display 45. Then, the microprocessor 50 terminates the subroutine and returns to the main routine.

In STEP S15, it is determined whether time to wait for the reception of the reply command pulse PL' has been elapsed. This waiting time may be set as appropriate, but in the present embodiment, it is set as 50 ms from transmission of the command pulse PL in STEP S12. When the waiting time of 50 ms is not yet elapsed, the microprocessor 50 returns to STEP S13 and confirms the reception of the reply command pulse PL' again. Thus, STEP S13 and STEP S15 are repeated until the reply command pulse PL' is confirmed in STEP S13 or passage of the waiting time of 50 ms is confirmed in STEP S15. Meanwhile, when it has been confirmed in STEP S15 that the waiting time of 50 ms has been elapsed, the microprocessor 50 determines that the storage device 71 is not connected to the main unit 41 and proceeds to STEP S16.

In STEP S16, the microprocessor 50 clears the connection flag and displays a mark or the like which indicates non-connection of the storage device 71 on the display 45. Then, the microprocessor 50 proceeds to STEP S17.

In STEP S17, the microprocessor 50 controls the electric power source circuit 58 so as to disconnect the electric power supply line V1 from the battery 57. Thereafter, the microprocessor 50 terminates the subroutine and returns to the main routine.

The above connection confirmation process is included in the main routine of the control program in the present embodiment and is executed either by turning on the main unit 41 or by completing calculation of the body fat percentage. However, only either one of these conditions may be taken as the condition for starting the execution of the process. Alternatively, the process may be a subroutine which can interrupt the main routine at the user' s option. That is, the process can be, for example, an interrupt subroutine which is executed when a user presses down a special switch among the operation switches 43 of the main unit 41 which is provided specifically for directing transmission of data to the storage device 71.

Although the second embodiment of the present invention has been described with referring to the drawings, the present invention does not have to be limited to this embodiment. To say nothing of modifications and applications which have already been discussed, a variety of other modifications and applications are also possible as long as they do not deviate from the constitutions described in claims.

For example, use of the data transmitter of the present invention is not limited to a part of the storage device incorporating the memory for data storage, and the data transmitter can be widely used in devices which need to conduct data transmissions corresponding to different data transmission capacities with multiple host devices having the data transmission capacities. In particular, standards for the connection terminals and data transmissions are not limited to USB, and a variety of other standards including RS-232C, RS-422 and IEEE1394 can also be used. Further, 3 or more transmission standards can be used. In addition, as data selecting means, switching of a software-based logic gate or a mechanical switching device operated directly by a user may also be used in addition to the analog switch.

Further, use of the storage device of the present invention is not limited to a part of the biological data acquiring apparatus. The storage device can be widely used as a general-purpose storage device incorporating a memory for data storage.

Furthermore, the biological data acquiring apparatus of the present invention is not limited to an apparatus which measures bioelectrical impedance between the bottoms of both feet of a user and can be widely used as an apparatus which measures bioelectrical impedance between both hands, between a hand and a foot or between other specific parts of a user. Further, the biological data acquiring apparatus of the present invention is not limited to a biological data acquiring apparatus of the type which measures the body weight or bioelectrical impedance of a user and can be widely used, for example, as an apparatus which measures or calculates a thickness of fat or a bone density by use of ultrasonic wave or the like or various other known apparatuses for acquiring biological data such as a blood pressure meter, a pulse meter, a pedometer and a consumed calorie meter. In addition, the biological data acquiring apparatus of the present invention is not limited to a stationary biological data acquiring apparatus and can be widely used as a portable biological data acquiring apparatus as well.

As described above, the storage device for a biological data acquiring apparatus according to the present invention is independent and detachable from the main unit of the biological data acquiring apparatus that incorporates biological data acquiring means for acquiring biological data and has biological data storing means for storing the biological data acquired by the biological data acquiring means. Thus, the storage device can be detached from the main unit of the biological data acquiring apparatus with the biological data stored in the biological data storing means and can be connected to a data processing terminal so as to transmit the stored biological data to the data processing terminal. Further, by accumulating the biological data in the data processing terminal or performing computations in the data processing terminal, desired biological data which are difficult to acquire by the biological data acquiring apparatus alone can be acquired.

Further, when the above storage device has program software for performing computations based on the biological data stored in the above biological data storing means, the program software can be executed in the data processing terminal when the storage device is connected to the data processing terminal. As a result, the need to store all biological data in the biological data storing means is obviated, and the storage capacity of the biological data storing means can be kept small, thereby making the storage device relatively inexpensive.

Alternatively, when the above storage device has program software for performing computations based on the biological data stored in the above biological data storing means and an operation element for executing the program software, the program software can be executed in the storage device, and biological data resulting from the execution of the software can be transmitted to the data processing terminal. As a result, the need to store all biological data in the biological data storing means is obviated, and the storage capacity of the biological data storing means can be kept small, thereby making the storage device relatively inexpensive.

Meanwhile, when the above storage device has a data transmitter which carries out data transmission with a host device via a connection terminal that accommodates a predetermined data transmission standard and which comprises data signal processing means for processing a number of different data signals including a data signal that accommodates the above predetermined data transmission standard and data signal selecting means for selecting a data signal to be processed by the data signal processing means, the storage device can be used between a host device (data processing terminal) which accommodates the above predetermined data transmission standard and a host device (biological data acquiring apparatus) which does not accommodate the standard, without increasing the number of the connection terminal.

When the above data signal selecting means selects the data signal based on a given identification signal transmitted from the host device, selection of the data signal can be made simply by connecting the storage device to the host device. Thus, the storage device can be used easily.

Further, when the above predetermined data transmission standard is the USB standard, the storage device can also conduct data transmission with a host device which does not accommodate the USB standard by use of a data transmitter which accommodates the widely prevalent USB standard.

Further, even when the above main unit of the biological data acquiring apparatus is stationary, the usability of the biological data acquiring apparatus can be improved because there is no need to move the main unit in transmitting biological data to the data processing terminal.

Alternatively, the biological data acquiring apparatus according to the present invention comprises a main unit which incorporates biological data acquiring means for acquiring biological data and a storage device which is independent and detachable from the main unit and has biological data storing means for storing the biological data acquired by the above biological data acquiring means. Thus, the storage device can be detached from the main unit with the biological data stored in the biological data storing means and can be connected to a data processing terminal so as to transmit the stored biological data to the data processing terminal. Further, by accumulating the biological data in the data processing terminal or performing computations in the data processing terminal, desired biological data which are difficult to acquire by the biological data acquiring apparatus alone can be acquired.

Further, when the above storage device has program software for performing computations based on the biological data stored in the above biological data storing means, the program software can be executed in the data processing terminal when the storage device is connected to the data processing terminal. As a result, the need to store all biological data in the biological data storing means is obviated, and the storage capacity of the biological data storing means can be kept small, thereby making the biological data acquiring apparatus relatively inexpensive.

Alternatively, when the above storage device has program software for performing computations based on the biological data stored in the above biological data storing means and an operation element for executing the program software, the program software can be executed in the storage device, and biological data resulting from the execution of the software can be transmitted to the data processing terminal. As a result, the need to store all biological data in the biological data storing means is obviated, and the storage capacity of the biological data storing means can be kept small, thereby making the biological data acquiring apparatus relatively inexpensive.

Meanwhile, when the above storage device has a data transmitter which carries out data transmission with a host device via a connection terminal that accommodates a predetermined data transmission standard and which comprises data signal processing means for processing a number of different data signals including a data signal that accommodates the above predetermined data transmission standard and data signal selecting means for selecting a data signal to be processed by the data signal processing means, data transmission can be carried out between the data processing terminal which accommodates the above predetermined data transmission standard and the biological data acquiring apparatus which does not accommodate the standard by use of the storage device, without increasing the number of the connection terminal of the storage device.

When the above data signal selecting means selects the data signal based on a given identification signal transmitted from the host device, selection of the data signal can be made simply by connecting the storage device to the host device. Thus, the storage device can be used easily.

Further, when the above predetermined data transmission standard is the USB standard, the storage device can also conduct data transmission with a host device which does not accommodate the USB standard by use of a data transmitter which accommodates the widely prevalent USB standard.

Further, even when the above main unit is stationary, the biological data acquiring apparatus can be of good usability because there is no need to move the main unit in transmitting biological data to the data processing terminal.

Alternatively, the data transmitter according to the present invention is a data transmitter which carries out data transmission with a host device via a connection terminal that accommodates a predetermined data transmission standard and which comprises data signal processing means for processing a number of different data signals including a data signal that accommodates the predetermined data transmission standard and data signal selecting means for selecting a data signal to be processed by the data signal processing means. Thus, the data transmitter can carry out data transmission by selecting a data signal of the predetermined data transmission standard when the host device accommodates the standard and by selecting a data signal of other standard when the host device does not accommodate the predetermined data transmission standard, without increasing the number of the connection terminal.

When the above data signal selecting means selects the data signal based on a given identification signal transmitted from the host device, selection of the data signal can be made simply by connecting the data transmitter to the host device. Thus, the data transmitter can be used easily.

Further, when the above predetermined data transmission standard is the USB standard, data transmission can be carried out with a host device which does not accommodate the USB standard by use of the data transmitter which accommodates the widely prevalent USB standard.

Further, the storage device according to the present invention comprises the above data transmitter according to the present invention and a memory for storing data. Thus, the storage device can be used between a host device which accommodates the above predetermined data transmission standard and a host device which does not accommodate the standard, without increasing the number of the connection terminal.

Further, the biological data acquiring apparatus according to the present invention is a biological data acquiring apparatus comprising a main unit which incorporates biological data acquiring means for acquiring biological data and a storage device which is independent from the main unit and detachable from the main unit via a terminal for data transmission and comprises the data transmitter according to the present invention and a memory for storing the biological data acquired by the above main unit. Thus, data transmission can be carried out between the data processing terminal which accommodates the above predetermined data transmission standard and the biological data acquiring apparatus which does not accommodate the standard by use of the storage device, without increasing the number of the connection terminal of the storage device.

Further, when the above storage device has program software for performing computations based on the biological data stored in the above memory, the program software can be executed in the data processing terminal when the storage device is connected to the data processing terminal. As a result, the need to store all biological data in the memory is obviated, and the storage capacity of the memory can be kept small, thereby making the biological data acquiring apparatus relatively inexpensive.

Alternatively, when the above storage device has program software for performing computations based on the biological data stored in the above memory and an operation element for executing the program software, the program software can be executed in the storage device, and biological data resulting from the execution of the software can be transmitted to the data processing terminal. As a result, the need to store all biological data in the memory is obviated, and the storage capacity of the memory can be kept small, thereby making the biological data acquiring apparatus relatively inexpensive.

Further, even when the above main unit is stationary, the biological data acquiring apparatus can be of good usability because there is no need to move the main unit in transmitting biological data to the data processing terminal.

## Claims

1. A storage device for a biological data acquiring apparatus, which is independent and detachable from the main unit of the biological data acquiring apparatus that incorporates biological data acquiring means for acquiring biological data and has biological data storing means for storing the biological data acquired by the biological data acquiring means,
wherein the storage device has a data transmitter which carries out data transmission with a host device via a connection terminal that accommodates a predetermined data transmission standard and which comprises data signal processing means for processing a number of different data signals including a data signal that accommodates the predetermined data transmission standard and data signal selecting means for selecting a data signal to be processed by the data signal processing means.

2. The device of claim 1, wherein the data signal selecting means selects the data signal based on a given identification signal transmitted from the host device.

3. The device of claim 1 or 2, wherein the predetermined data transmission standard is the USB standard.

4. The device of any one of claims 1 to 3, wherein the main unit of biological data acquiring apparatus is stationary.

5. The storage device of any one of claims 1 to 4, wherein the number of different data signals is a number of different kinds of data signals.

6. A biological data acquiring apparatus comprising:
a main unit incorporating biological data acquiring means for acquiring biological data and
a storage device being independent and detachable from the main unit and having biological data storing means for storing the biological data acquired by the biological data acquiring means,
wherein the storage device has a data transmitter which carries out data transmission with a host device via a connection terminal that accommodates a predetermined data transmission standard and which comprises data signal processing means for processing a number of different data signals including a data signal that accommodates the predetermined data transmission standard and data signal selecting means for selecting a data signal to be processed by the data signal processing means.

7. The apparatus of claim 6, wherein the data signal selecting means selects the data signal based on a given identification signal transmitted from the host device.

8. The apparatus of claim 6 or 7, wherein the predetermined data transmission standard is the USB standard.

9. The apparatus of any one of claims 6 to 8, wherein the main unit of the biological data acquiring apparatus is stationary.

10. The apparatus of any one of claims 6 to 9, wherein the number of different data signals is a number of different kinds of data signals.
